# EUROPEAN PATENT APPLICATION

(11) **EP 3 243 541 A1**
(43) Date of publication of application: **15.11.2017**
(21) Application number: 15876497.7
(22) Date of filing: 20.01.2015
(51) Int. Cl.: A61M 5/32, A61M 5/178

(54) **SYRINGE**

(30) Priority: 05.01.2015 CN 201510002629
(71) Applicant: Minggao Meditech (Kunshan) Co., Ltd., Suzhou, Jiangsu 201103 (CN)
(72) Inventor: WANG, Wenpin, Taichung City Taiwan 20243 (TW)
(74) Representative: Boden, Keith McMurray
(86) International application number: PCT/CN2015/071070
(87) International publication number: WO 2016/109989

(57) **Abstract**

The present invention provides a syringe including a needle holder, an inner sliding sleeve and a plunger; wherein, the needle holder is provided for arranging a injecting needle; the plunger, which is provided for pushing liquid medicine into the needle holder, is arranged at an opposite position with the needle holder; the needle holder is embedded in and connected to the inner sliding sleeve. The needle holder and the inner sliding sleeve can slide relatively to each other. Before an injection, the inner sliding sleeve slides, and the inner sliding sleeve and the needle holder are overlapped, to expose the injecting needle for the injection. After the injection, the inner sliding sleeve slides back, so that the injecting needle is packed into the inner sliding sleeve. The syringe of the present invention, after the injection, the inner sliding sleeve slides towards the same direction of the needle injecting, so that the injecting needle is packed into the inner sliding sleeve, to avoid inserting someone's body and infecting disease because of the needle exposed to the air.

## Description

### FIELD OF THE PRESENT INVENTION

The present invention relates to a field of medical equipment, especially a syringe.

### BACKGROUD OF THE PRESENT INVENTION)

In prior art, after an injection, an injecting needle of a syringe is easily stained by a patient's blood. The injecting needle may insert into someone's body if it isn't sequestered immediately. Therefore, injuries to the body will happen, and meanwhile, the patient's blood remaining at the injecting needle can also lead to disease infections.

### SUMMARY OF THE PRESENT INVENTION

The present invention provides a syringe in order to avoid injury resulted in exposure of the injecting needle, when the syringe has been operated.

The syringe of the present invention includes a needle holder, a first sliding sleeve and a plunger.

Wherein, the needle holder is provided for arranging an injecting needle. The plunger, which is provided for pushing liquid medicine into the needle holder, is arranged at an opposite position to the needle holder. The needle holder is embedded in and connected to the first sliding sleeve; the needle holder and is the first sliding sleeve can slide relatively to each other. Before an injection, the first sliding sleeve slides to overlap the needle holder. The injecting needle is exposed for the injection. After the injection, the first sliding sleeve slides back so that the injecting needle is packed into the first sliding sleeve.

Preferably, a first bulge part and an elastic extending part are provided to the needle holder. The first bulge part is arranged on the tail end of the needle holder through the elastic extending part. A first positioning groove and a second positioning groove, which are matched with the first bulge part, are arranged on the inside wall of first sliding sleeve. The first positioning groove is arranged on the head end of the first sliding sleeve. The second positioning groove is arranged on the tail end of the inner sliding sleeve.

Preferably, a first guiding groove, which extends along with the first sliding sleeve, is arranged on inside the wall of the first sliding sleeve. A first guiding bulge part, which is matched with the first guiding groove, is arranged on outside wall of the needle holder.

Preferably, a first slope surface, which is matched with the first bulge part, is arranged on the edge of the tail end of inside wall of the first sliding sleeve.

Preferably, a first inner sliding wall, which is matched with the first bulge part, is arranged on inside wall of the first sliding sleeve. The first inner sliding wall is located at one side, towards the first positioning groove, of the second positioning groove. And the height of the first inner sliding wall decreases gradually from the second positioning groove to the first positioning groove.

Preferably, a third inner sliding wall, which is matched with the first bulge part, is arranged on inside wall of the first sliding sleeve. The third inner sliding wall is located at one side, towards the second positioning groove, of the first positioning groove. The height of the third inner sliding wall decreases gradually from the first positioning groove to the second positioning groove. The angle between the first inner sliding wall and inside wall of the first sliding sleeve is greater than the angle between the third inner sliding wall and inside wall of the first sliding sleeve.

Preferably, the syringe also includes a second sliding sleeve.

Wherein, the first sliding sleeve is embedded in and connected to the second sliding sleeve, the second sliding sleeve and the inner sliding sleeve can slide relatively to each other. Before the injection, the first sliding sleeve and the second sliding sleeve slid and the inner sliding sleeve, the outer sliding sleeve and the needle holder are overlapped. The injecting needle is exposed for the injection. After the injection, the first sliding sleeve and the second sliding sleeve slide back, so that the injecting needle is hided into the first sliding sleeve and the second sliding sleeve.

Preferably, a second bulge part is arranged on the head end of outside wall of the first sliding sleeve. A third positioning groove and a fourth positioning groove, which are matched with the second bulge part, are arranged on the inside wall of second sliding sleeve. The third positioning groove is arranged on the head end of the second sliding sleeve. The fourth positioning groove is arranged on the tail end of the outer sliding sleeve.

Preferably, a second guiding groove, which extends along with the second sliding sleeve, is arranged on inside wall of the second sliding sleeve. A second guiding bulge part, which is matched with the second guiding groove, is arranged on outside wall of the first sliding sleeve.

Preferably, a second slope surface, which is matched with the second bulge part, is arranged on the edge of the tail end of inside wall of the second sliding sleeve.

Preferably, a second inner sliding wall, which is matched with the second bulge part, is arranged on inside wall of the second sliding sleeve. The second inner sliding wall is located at one side, towards the third positioning groove, of the fourth positioning groove. And the height of the second inner sliding wall decreases gradually from the fourth positioning groove to the third positioning groove.

Preferably, a fourth inner sliding wall, which is matched with the second bulge part, is arranged on inside wall of the second sliding sleeve. The fourth inner sliding wall is located at one side, towards the forth positioning groove, of the third positioning groove. And the height of the fourth inner sliding wall decreases gradually from the third positioning groove to the fourth positioning groove. And the angle between the second inner sliding wall and the inside wall of the second sliding sleeve is greater than the angle between the fourth inner sliding wall and the inside wall of the second sliding sleeve.

Preferably, the syringe also includes a piston, where a liquid-guiding channel is arranged. The liquid-guiding channel comprises, successively, an injecting channel, a liquid-diverging hole and a guiding channel. The piston is inserted in a chamber of the needle holder.

The syringe of the present invention, before the injection, the first sliding sleeve slides towards an opposite direction of the injecting direction of the injecting needle. Thus the injecting needle is exposed for the injection. After the injection, the first sliding sleeve slides towards the same direction of the injection direction of the injecting needle. Therefore, the needle is packed into the first sliding sleeve, so that a stab on someone's body pierced by the exposed injecting needle and the disease infection resulted from the stab can be avoided.

### DESCRIPTION OF THE FIGURES

The figures mentioned in the embodiments of present invention will be described briefly so that the technical solution in the embodiments can be comprehended more clearly. Obviously, the figures are described just for some special embodiments of the present invention. Moreover, some other figures according to these figures can be obtained by ordinary skilled person in the art, with uncreative work.
Fig. 1 shows a view of the syringe of the first embodiment during an injection.
Fig. 2 shows a view of the syringe of the first embodiment after the injection.
Fig. 3 shows a part view of the syringe of the first embodiment when during the injection.
Fig. 4 shows a part view of the syringe of the first embodiment after the injection.
Fig. 5 shows a perspective view of the needle holder of the first embodiment.
Fig. 6 shows a side cross-sectional view of the needle holder of the first embodiment.
Fig. 7 shows a perspective view of the inner sliding sleeve of the first embodiment.
Fig. 8 shows a cross-sectional view of the inner sliding sleeve of the first embodiment.
Fig. 9 shows a side cross-sectional view of the inner sliding sleeve of the first embodiment.
Fig. 10 shows a perspective view of the outer sliding sleeve of the first embodiment.
Fig. 11 shows a part cross-sectional view of the outer sliding sleeve of the first embodiment.
Fig. 12 shows a side cross-sectional view of the outer sliding sleeve of the first embodiment.
Fig. 13 shows a part cross-sectional view of the connection between the needle holder and the inner sliding sleeve of the first embodiment.
Fig. 14a-d respectively shows a side cross-sectional view of the connection between the needle holder and the inner sliding sleeve of the first embodiment.
Fig. 15 shows a part cross-sectional view of the connection among the needle holder, the inner sliding sleeve and the outer sliding sleeve of the first embodiment.
Fig. 16a-d respectively shows a side cross-sectional view of the connection among the needle holder, the inner sliding sleeve and the outer sliding sleeve of the first embodiment.
Fig. 17 shows a perspective view of the needle holder and the piston of the first embodiment.
Fig. 18 shows a side cross-sectional view of the piston of the first embodiment.
Fig. 19 shows a part cross-sectional view of the connection among the needle holder, the inner sliding sleeve and the outer sliding sleeve of the second embodiment.
Fig. 20 shows a side cross-sectional view of the connection between the inner sliding sleeve and the needle holder of the second embodiment.
Fig. 21 shows a side cross-sectional view of the connection among the inner sliding sleeve, the outer sliding sleeve and the needle holder of the second embodiment.
Fig. 22 shows a part cross-sectional view of temporary positioning condition of the connection among the inner sliding sleeve, the outer sliding sleeve and the needle holder of the second embodiment.
Fig. 23 shows a view of syringe of the third embodiment.

Description of reference signs:
1. needle holder, 101. first bulge part, 102. elastic extending part, 103. first guiding bulge part, 104. chamber;
2. inner sliding sleeve, 201. second bulge part, 202. second positioning groove, 203. first guiding groove, 204. second bulge guiding part, 205. first hollow structure, 206. first slope surface, 207. first positioning groove, 208. first inner sliding wall, 209. third inner sliding wall;
3. outer sliding sleeve, 301. third positioning groove, 302. fourth positioning groove, 303. second slope surface, 304. second guiding groove, 305. second hollow structure, 306. hand-held part, 307. second inner sliding wall, 308. fourth inner sliding wall;
4. plunger;
5. injecting needle.
6. piston, 603. injecting channel, 602. liquid-diverging hole, 601. guiding channel.

### EMBODIMENTS

Referring to the figures, the technical solution of the present invention will be described more clearly and fully, so that the objects, technical solutions and advantages can be easily understood. Obviously, the mentioned embodiments just partly, but not completely, show examples of the present invention. Some other examples can be obtained by ordinary skilled person in the art based on the disclosure of the embodiments of the present invention with uncreative work. And the other examples also fall into the scope of the present invention.

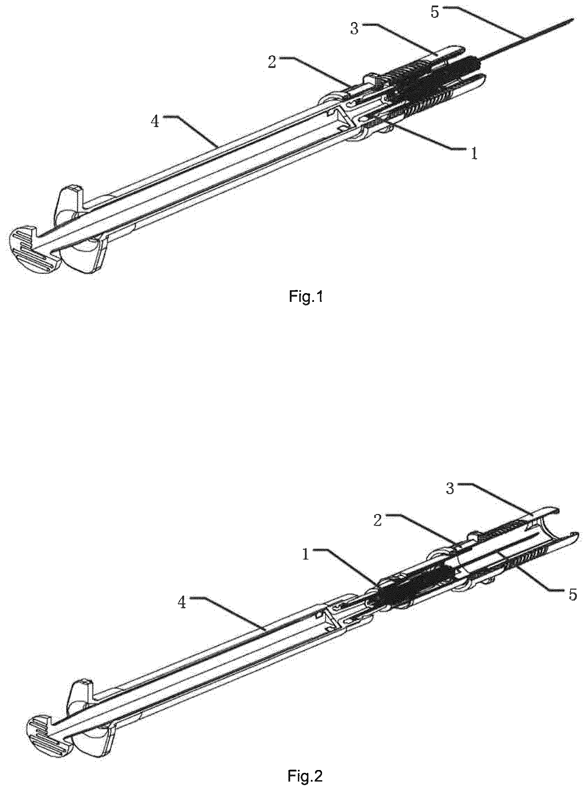

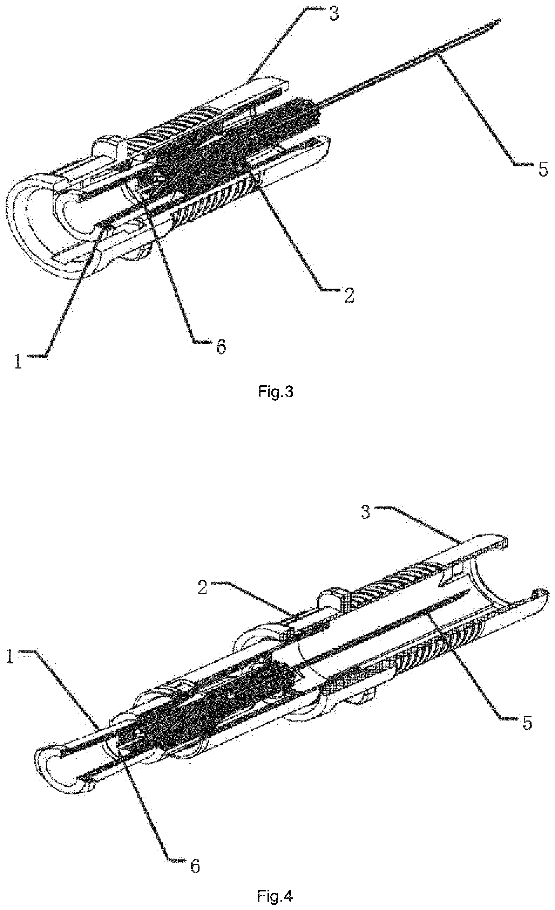

Fig. 1 shows a view of the syringe of the first embodiment during an injection. Fig. 2 shows a view of the syringe of the first embodiment after the injection. Fig. 3 shows a part view of the syringe of the first embodiment during the injection. Fig. 4 shows a part view of the syringe of the first embodiment after the injection. As shown in figure 1, figure 2, figure 3 and figure 4, the syringe of the first embodiment includes NEEDLE HOLDER 1, INNER SLIDING SLEEVE 2, OUTER SLIDING SLEEVE 3 AND PLUNGER 4. The NEEDLE HOLDER 1 is used for arranging INJECTING NEEDLE 5. The PLUNGER 4, which is used for adding liquid medicine into the NEEDLE HOLDER 1, is arranged on the opposite position with the NEEDLE HOLDER 1. The INNER SLIDING SLEEVE 2 is embedded in and connected to the OUTER SLIDING SLEEVE 3. The INNER SLIDING SLEEVE 2 and the OUTER SLIDING SLEEVE 3 can slide relatively to each other. The NEEDLE HOLDER 1 is embedded in and connected to the INNER SLIDING SLEEVE 2. The NEEDLE HOLDER 1 and the INNER SLIDING SLEEVE 2 can slide relatively to each other. Before an injection, the INNER SLIDING SLEEVE 2 and the OUTER SLIDING SLEEVE 3 slide to overlap the INNER SLIDING SLEEVE 2 and the OUTER SLIDING SLEEVE 3 outside the NEEDLE HOLDER 1. So that the INJECTING NEEDLE 5 can be exposed for the injection. After the injection, the INNER SLIDING SLEEVE 2 and the OUTER SLIDING SLEEVE 3 slide back. Then the INJECTING NEEDLE 5 is packed into the inner sliding sleeve 2 and the OUTER SLIDING SLEEVE 3.

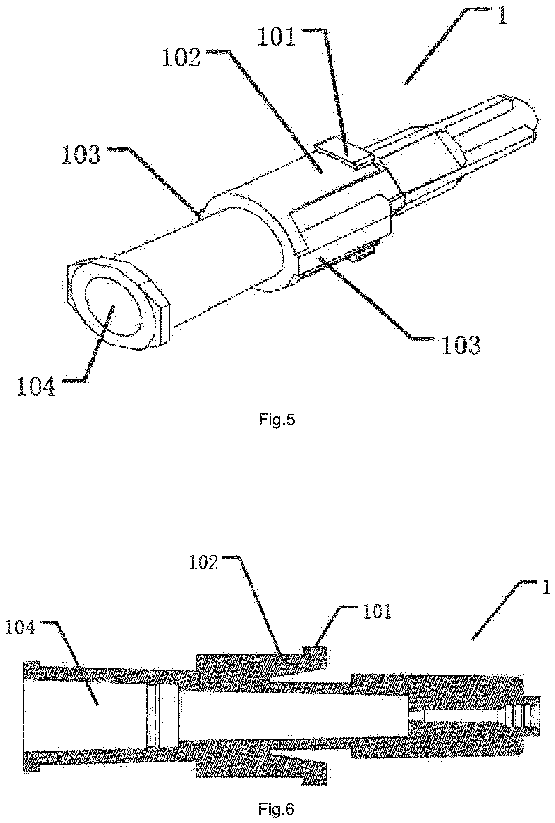

Fig. 5 shows a perspective view of the needle holder of the first embodiment. Fig. 6 shows a side cross-sectional view of the needle holder of the first embodiment. As shown in the figure 5 and figure 6, the FIRST BULGE PART 101 and the ELASTIC EXTENDING PART 102 are arranged on the NEEDLE HOLDER 1. The FIRST BULGY PART 101 is arranged on the tail end of the NEEDLE HOLDER 1 through the ELASTIC EXTENDING PART 102. The FIRST GUIDING BULGE PART 103, which is matched with the first guiding groove of the INNER SLIDING SLEEVE 2, arranged on the NEEDLE HOLDER 1. The CHAMBER 104 is arranged in the NEEDLE HOLDER 1.

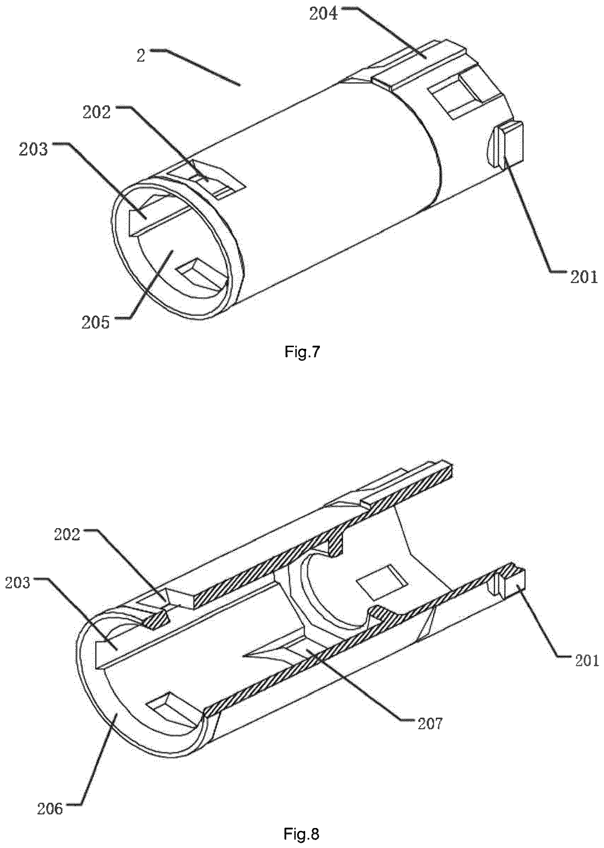

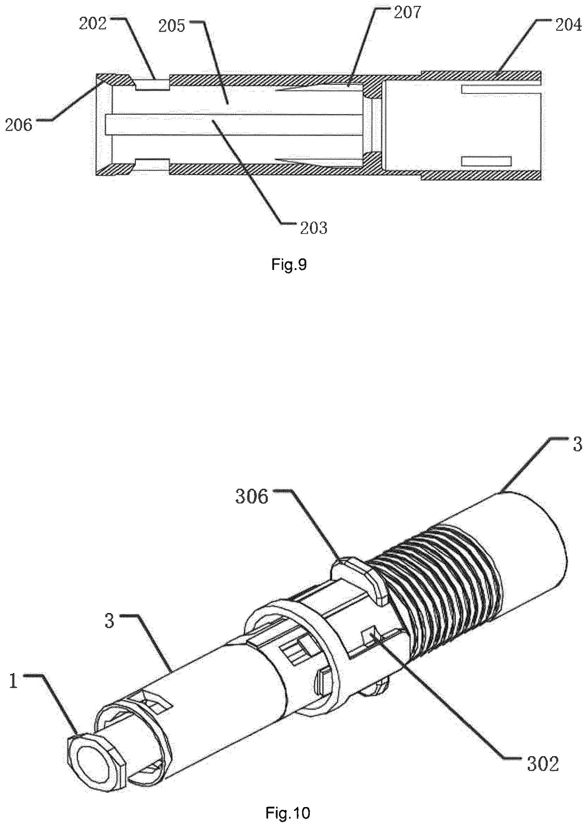

Fig. 7 shows a perspective view of the inner sliding sleeve of the first embodiment. Fig. 8 shows a cross-sectional view of the inner sliding sleeve of the first embodiment. Fig. 9 shows a side cross-sectional view of the inner sliding sleeve of the first embodiment. As shown in the figure 7, figure 8 and figure 9, the FIRST POSITIONING GROOVE 207 and the SECOND POSITIONING GROOVE 202, which are matched with the FIRST BULGE PART 101 of the NEEDLE HOLDER 1, are arranged on the INNER SLIDING SLEEVE 2. The FIRST POSITIONING GROOVE 207 is arranged on the head end of inside wall of the INNER SLIDING SLEEVE 2. A FIRST GUIDING GROOVE 203, which extends along with the INNER SLIDING SLEEVE 2, is arranged on inside wall of the INNER SLIDING SLEEVE 2. The FIRST GUIDING GROOVE 203 is matched with the FIRST GUIDING BULGE PART 103 of outside wall of the NEEDLE HOLDER 1. A FIRST SLOPE SURFACE 206, which is matched with the FIRST BULGE PART 101 of the NEEDLE HOLDER 1, is arranged on the edge of the tail end of inside wall of the INNER SLIDING SLEEVE 2. A SECOND BULGE PART 201 is arranged on the head end of the outside wall of the INNER SLIDING SLEEVE 2.

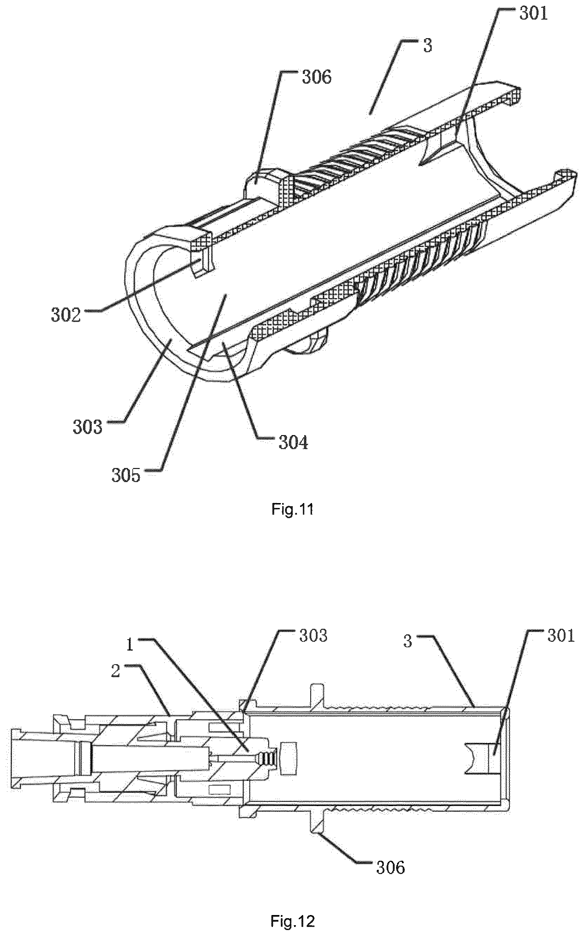

Fig. 10 shows a perspective view of the outer sliding sleeve of the first embodiment. Fig. 11 shows a part cross-sectional view of the outer sliding sleeve of the first embodiment. Fig. 12 shows a side cross-sectional view of the outer sliding sleeve of the first embodiment. As shown in the figure 10, figure 11 and figure 12, a THIRD POSITIONING GROOVE 301 and a FOURTH POSITIONING GROOVE 302, which are matched with the SECOND BULGE PART 201, are arranged on the OUTER SLIDING SLEEVE 3. The THIRD POSITIONING GROOVE 301 is arranged on the head end of inside wall of the OUTER SLIDING SLEEVE 3. The FOURTH POSITIONING GROOVE 302 is arranged on the tail end of inside wall of the OUTER SLIDING SLEEVE 3. A SECOND GUIDING GROOVE 304, which extends along with the OUTER SLIDING SLEEVE 3, is arranged on inside wall of the OUTER SLIDING SLEEVE 3. The SECOND GUIDING GROOVE 304 is matched with the SECOND GUIDING BULGE PART 204 of the INNER SLIDING SLEEVE 2. Inside the OUTER SLIDING SLEEVE 3 a SECOND HOLLOW STRUCTURE 305 forms. A HAND-HELD PART 306 is arranged outside the OUTER SLIDING SLEEVE 3. A SECOND SLOPE SURFACE 303, which is matched with the SECOND BULGE PART 201, is arranged on the edge of the tail end of inside wall of the OUTER SLIDING SLEEVE 3. As shown in the figure 7, a SECOND GUIDING BULGE PART 204, which is matched with the SECOND GUIDING GROOVE 304, is arranged on outside wall of the INNER SLIDING SLEEVE 2. The SECOND BULGE PART 201 is arranged on the head end of outside wall of the INNER SLIDING SLEEVE 2.

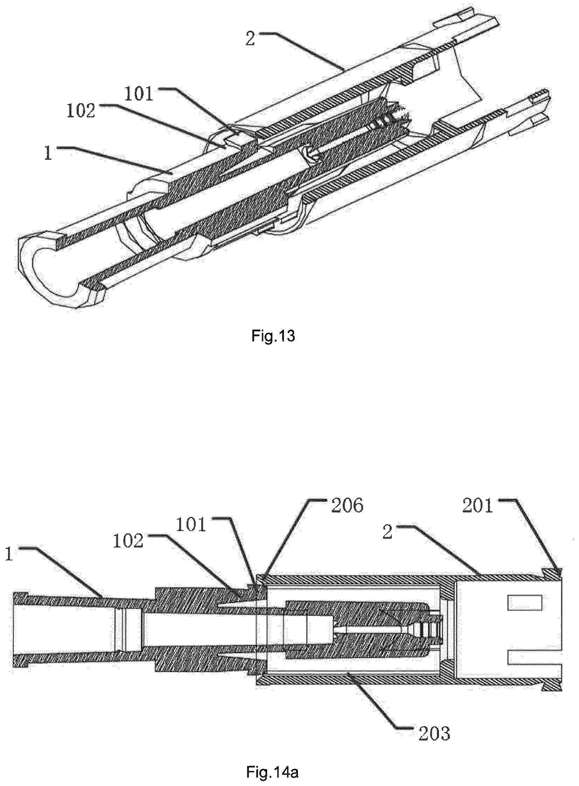

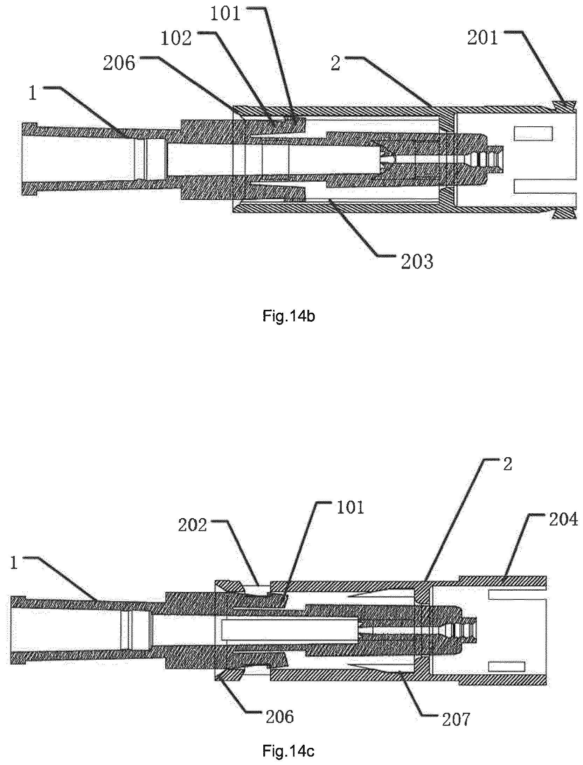

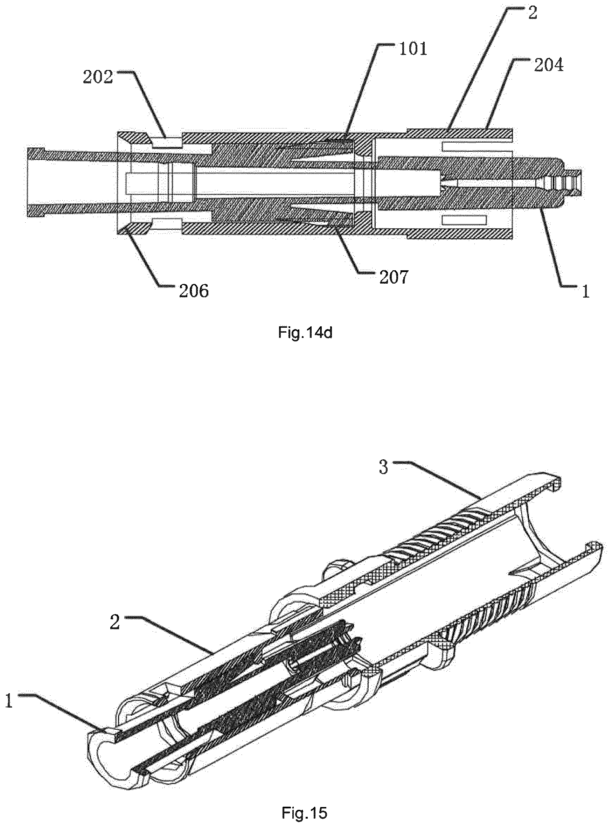

Fig. 13 shows a part cross-sectional view of the connection between the needle holder and the inner sliding sleeve of the first embodiment. Fig. 14a-d respectively shows a side cross-sectional view of the connection between the needle holder and the inner sliding sleeve of the first embodiment. As shown in the figure 13, figure 14a-d, the FIRST BULGE PART 101 of the NEEDLE HOLDER 1 contacts with the first SLOPE SURFACE 206 of the INNER SLIDING SLEEVE 2, pushing the NEEDLE HOLDER 1 into the FIRST HOLLOW STRUCTURE 205 of the INNER SLIDING SLEEVE 2. Because the ELASTIC EXTENDING PART 102 has elasticity, the FIRST BULGE PART 101 is squeezed by the INNER SLIDING SLEEVE 2 through the ELASTIC EXTENDING PART 102 towards a center of the NEEDLE HOLDER 1. The FIRST BULGE PART 101 goes across the SECOND POSITIONING GROOVE 202. Rotating the NEEDLE HOLDER 1 around it's center axis line, so that the FIRST GUIDING BULGE PART 103 enters the FIRST POSITIONING GROOVE 207, and pushes the NEEDLE HOLDER 1 into the INNER SLIDING SLEEVE 2. The FIRST BULGY PART 101 enters the FIRST POSITIONING GROOVE 207, and the NEEDLE HOLDER 1 is fixed into the INNER SLIDING SLEEVE 2, so that the INNER SLIDING SLEEVE 2 overlaps with the NEEDLE HOLDER 1. The injection can be operated.

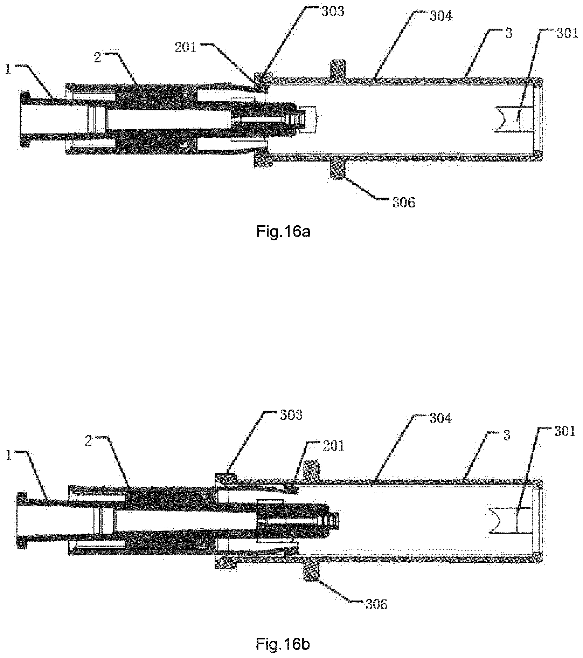

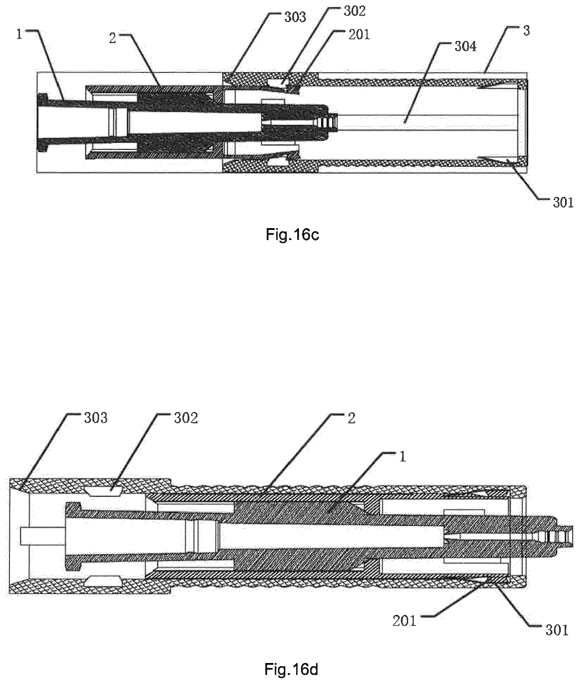

Fig. 15 shows a part cross-sectional view of the connection among the needle holder, the inner sliding sleeve and the outer sliding sleeve of the first embodiment. Fig.16a-d respectively shows a side cross-sectional view of the connection among the needle holder, the inner sliding sleeve and the outer sliding sleeve of the first embodiment. As shown in the figure 15, figure 16a-d, the SECOND BULGE PART 201 of the INNER SLIDING SLEEVE 2 contacts with the SECOND BEVEL 303 of the OUTER SLIDING SLEEVE 3, pushing the INNER SLIDING SLEEVE 2 into the SECOND HOLLOW STRUCTURE 305 of the OUTER SLIDING SLEEVE 3, the SECOND BULGE PART 201 goes across the FOURTH POSITIONING GROOVE 302. Rotating the INNER SLIDING SLEEVE 2 around its center axis line. So that the SECOND GUIDING BULGE PART 204 enters the SECOND POSITIONING GROOVE 304 and pushes the INNER SLIDING SLEEVE 2 into the OUTER SLIDING SLEEVE 3. The SECOND BULGE PART 201 enters the THIRD POSITIONING GROOVE 301, and the INNER SLIDING SLEEVE 2 is fixed into the OUTER SLIDING SLEEVE 3, so that the NEEDLE HOLDER 1, the INNER SLIDING SLEEVE 2 and the OUTER SLIDING SLEEVE 3 overlap each other. Therefore the INJECTING NEEDLE 5 is exposed for the injection.

After the injection, pulling the NEEDLE HOLDER 1 out from the INNER SLIDING SLEEVE 2, pulling the INNER SLIDING SLEEVE 2 out from the OUTER SLIDING SLEEVE 3, so that the FIRST BULGY PART 101 enters the SECOND POSITIONING GROOVE 202 and the SECOND BULGY PART 201 enters the FOURTH POSITIONING GROOVE 302. Therefore the positions of the NEEDLE HOLDER 1, the INNER SLIDING SLEEVE 2 and the OUTER SLIDING SLEEVE are fixed relatively. The INJECTING NEEDLE 5 is packed into the INNER SLIDING SLEEVE 2 and the OUTER SLIDING SLEEVE 3, to avoid exposing the INJECTING NEEDLE 5 to the air or hurting someone's body by the INJECTING NEEDLE 5.

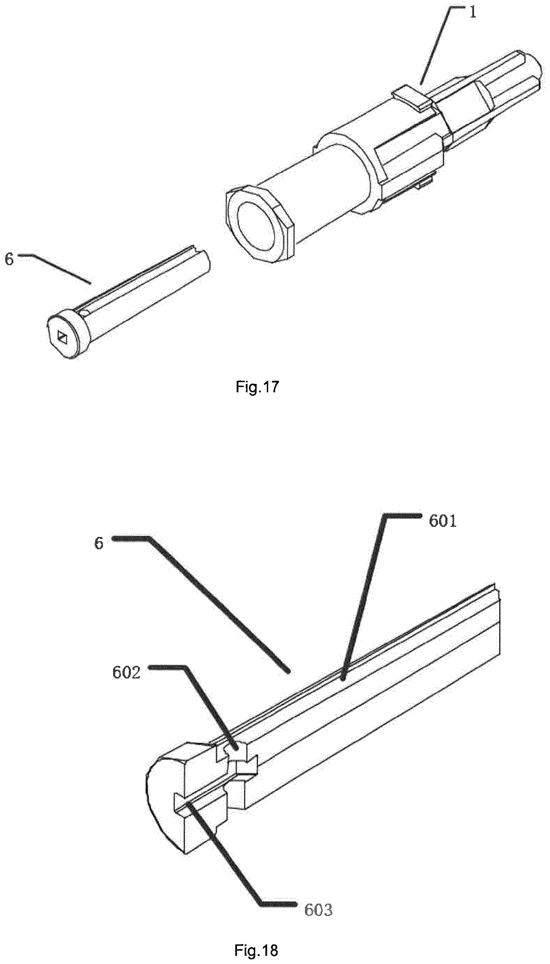

Fig. 17 shows a perspective view of the needle holder and the piston of the first embodiment. Fig. 18 shows a side cross-sectional view of the piston of the first embodiment. The syringe of the embodiment also includes PISTON 6 with a liquid-guiding channel. The piston 6 is inserted into the CHAMBER 104 of the NEEDLE HOLDER 1. The liquid-guiding channel includes INJECTING CHANNEL 603, LIQUID-DIVERGING HOLE 602, and GUIDING CHANNEL 601, successively. During the injection, the liquid flows through INJECTING CHANNEL 603, LIQUID-DIVERGING HOLE 602, and GUIDING CHANNEL 601, successively, and then the liquid is injected into a patient's body. The PISTON 6 can reduce the flow rate and the residue amount of the potion.

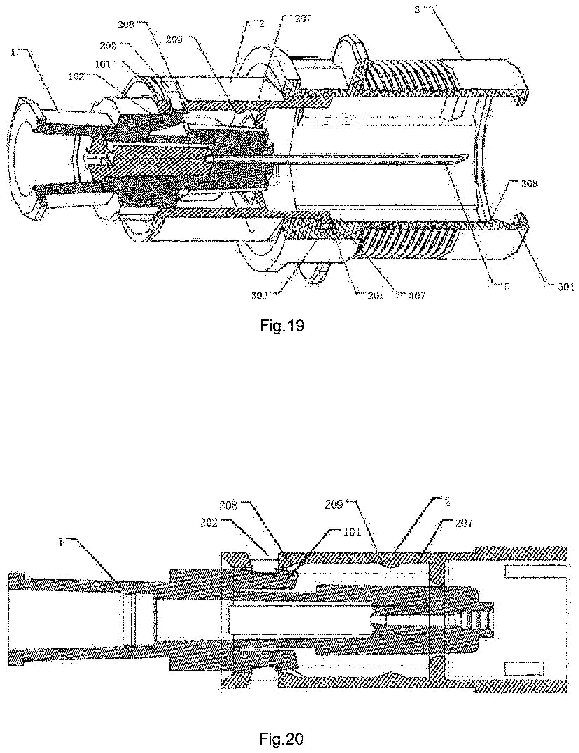

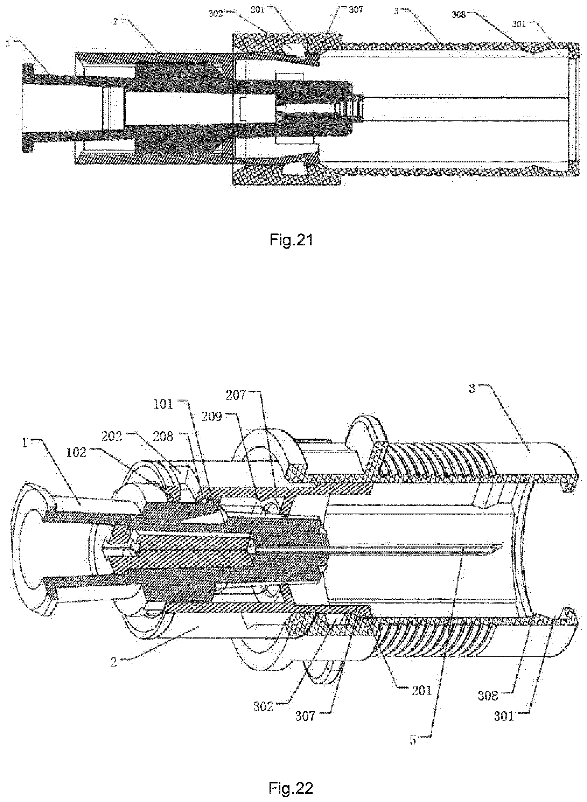

Fig. 19 shows a part cross-sectional view of the connection among the needle holder, the inner sliding sleeve and the outer sliding sleeve of the second embodiment. Fig. 20 shows a side cross-sectional view of the connection between the inner sliding sleeve and the needle holder of the second embodiment. Fig. 21 shows a side cross-sectional view of the connection among the inner sliding sleeve, the outer sliding sleeve and the needle holder of the second embodiment. As shown in the figure 19, figure 20 and figure 21, the structure of the syringe in the second embodiment is similar with the structure of the syringe in the first embodiment, expect for the following difference: a FIRST INNER SLIDING WALL 208 is arranged on the INNER SLIDING SLEEVE 2 and a SECOND INNER SLIDING WALL 307 is arranged on the OUTER SLIDING SLEEVE 3 in the second embodiment.

As shown in the figure 20, specifically, a FIRST INNER SLIDING WALL 208, which is matched with the FIRST BULGE PART 101, is arranged on the inside wall of the INNER SLIDING SLEEVE 2. The FIRST INNER SLIDING WALL 208 is located at one side, towards the FIRST POSITIONING GROOVE 207, of the SECOND POSITIONING GROOVE 202. And the height of the FIRST INNER SLIDING WALL 208 decreases gradually from the SECOND POSITIONING GROOVE 202 to the FIRST POSITIONING GROOVE 207. And the angle between the FIRST INNER SLIDING WALL 208 and the inside wall of the INNER SLIDING SLEEVE 2 is greater than the angel between the THIRD INNER SLIDING WALL 209 and the inside wall of the INNER SLIDING SLEEVE 2.

As shown in the figure 21, a SECOND INNER SLIDING WALL 307, which is matched with the SECOND BULGE PART 201, is arranged on the inside wall of the OUTER SLIDING SLEEVE 3, while the SECOND INNER SLIDING WALL 307 is located at the side, towards the THIRD POSITIONING GROOVE 301, of the FOURTH POSITIONING GROOVE 302. And the height of the SECOND INNER SLIDING WALL 307 decreases gradually from the THIRD POSITIONING GROOVE 301 to the FOURTH POSITIONING GROOVE 302. And the angle between the SECOND INNER SLIDING WALL 307 and the inside wall of the OUTER SLIDING SLEEVE 3 is greater than the angle between the FOURTH INNER SLIDING WALL 308 and the inside wall of the OUTER SLIDING SLEEVE 3.

Fig. 22 shows a part cross-sectional view of temporary positioning condition of the connection among the inner sliding sleeve, the outer sliding sleeve and the needle holder of the second embodiment. As shown in the figure 22, the first inner sliding wall 208 and the SECOND INNER SLIDING WALL 307 are used for fixing a temporary position before the injection. When the syringe is full of the liquid and a medical worker is ready to inject, if there is something suddenly happens and the injection is stopped, the INJECTING NEEDLE 5 may hurt someone if it is exposed to the air. In this situation, the INNER SLIDING SLEEVE 2 and the OUTER SLIDING SLEEVE 3 can be slid, so that the FIRST BULGE PART 101 is pressed at the FIRST INNER SLIDING WALL 208, and the SECOND BULGE PART 201 is pressed at the SECOND INNER SLIDING WALL 307, so the INNER SLIDING SLEEVE 2 and the OUTER SLIDING SLEEVE 3 are fixed temporarily, meanwhile the injecting needle 5 is packed by the INNER SLIDING SLEEVE 2 and the OUTER SLIDING SLEEVE 3 together, avoiding hurting someone's body by the needle is exposed to the air. Because the first bulge part 101 is not in the SECOND POSITIONING GROOVE 202, and the second bulgy part 201 is not in the FOURTH POSITIONING GROOVE 302 either, thus the positions of the INNER SLIDING SLEEVE 2 and the OUTER SLIDING SLEEVE 3 are not fixed completely. If the medical worker begins the injection, the INNER SLIDING SLEEVE 2 and the OUTER SLIDING SLEEVE 3 slide towards an opposite direction of the direction of the injection, to expose INJECTING NEEDLE 5 to the air. Therefore, the injection can be continued.

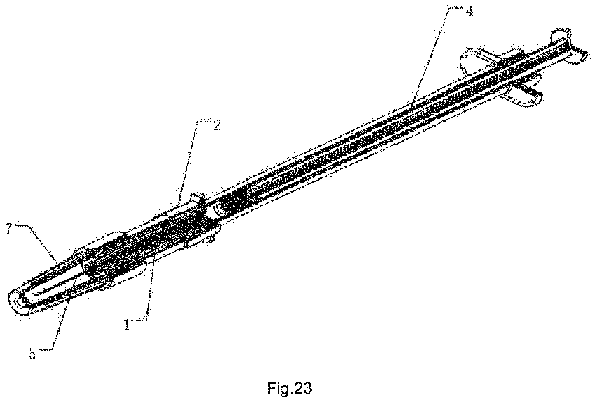

Fig. 23 shows a view of the syringe of the third embodiment, as shown in the figure 23, the syringe in the third embodiment includes NEEDLE HOLDER 1, INNER SLIDING SLEEVE 2 and PLUNGER 4. PROTECTING SLEEVE 7, which is used to protect the INJECTING NEEDLE 5, is arranged outside the NEEDLE HOLDER 1.

NEEDLE HOLDER 1 is used for arranging the NEEDLE 5. The PLUNGER 4, which is used for pushing liquid into the NEEDLE HOLDER 1, is arranged at the opposite position of the NEEDLE HOLDER 1. The NEEDLE HOLDER 1 is embedded in and connected to the INNER SLIDING SLEEVE 2, meanwhile NEEDLE HOLDER 1 and INNER SLIDING SLEEVE 2 can slide relatively to each other. Before the injection, the INNER SLIDING SLEEVE 2 slides, and the INNER SLIDING SLEEVE 2 and the NEEDLE HOLDER 1 are overlapped, to expose the INJECTING NEEDLE 5 for the injection. After the injection, the INNER SLIDING SLEEVE 2 slides back, to pack the INJECTING NEEDLE 5 into the INNER SLIDING SLEEVE 2. In the third embodiment, the NEEDLE HOLDER 1 as shown in the figures 5 to 6, the INNER SLIDING SLEEVE 2 as shown in the figures 7 to 9, and the PISTON 6 as shown in the figure 17 to 18 can be adopted for the syringe. Moreover, the FIRST INNER SLIDING WALL as shown in the figures 19 to 20 can also be arranged on the INNER SLIDING SLEEVE 2.

The syringe in the third embodiment is mainly provided for a shorter INJECTING NEEDLE 5. The shorter INJECTING NEEDLE 5 can be packed completely just by the INNER SLIDING SLEEVE 2. It is unnecessary to arrange the OUTER SLIDING SLEEVE. Therefore the syringe has a simplified structure and features materials saving.

At last, it should to be noted that the above-mentioned embodiments are just used for illustrating the technological solutions as some examples, but not limitation of the present invention. For the ordinary skilled person in the art, it should be understood that the some modifications for the technical solutions of the above-mentioned embodiments or some equivalent substitutions for part/entirety technical features of the technical solutions can be done; meanwhile the modifications and equivalent substitutions don't make the related technical solutions go beyond the spirit and scope of the present invention.

## Claims

1. A syringe includes a needle holder, an inner sliding sleeve and a plunger; wherein the needle holder is provided for arranging a injecting needle;
the plunger, which is provided for pushing liquid medicine into the needle holder, is arranged at the opposite position of the needle holder;
the needle holder is embedded in and connected to the inner sliding sleeve, the needle holder and the inner sliding sleeve can slide relatively to each other; before an injection, the inner sliding sleeve is slid and the inner sliding sleeve and the needle holder are overlapped, to expose the injecting needle for the injection; after the injection, the inner sliding sleeve is slid back, so that the injecting needle is packed into the inner sliding sleeve.

2. The syringe of claim 1, wherein a first bulge part and an elastic extending part are arranged on the needle holder;
the first bulge part is arranged on the tail end of the needle holder through the elastic extending part;
a first positioning groove and a second positioning groove, which are matched with
the first bulge part, are arranged on the inner sliding sleeve;
the first positioning groove is arranged on the head end of an inside wall of the inner sliding sleeve;
the second positioning groove is arranged on the tail end of an inside wall of the inner sliding sleeve.

3. The syringe of claim 2, wherein a first guiding groove, which extends along with the inner sliding sleeve, is arranged on the inside wall of the inner sliding sleeve; a first guiding bulge part, which is matched with the first guiding groove, is arranged on an outside wall of the needle holder.

4. The syringe of claim 3, wherein a first slope surface, which is matched with the first bulge part, is arranged on the edge of the tail end of the inside wall of the inner sliding sleeve.

5. The syringe of claim 3, wherein a first inner sliding wall, which is matched with the first bulge part, is arranged on the inside wall of the inner sliding sleeve;
the first inner sliding wall is located at one side, towards the first positioning groove, of the second positioning groove;
and the height of the first inner sliding wall decreases gradually from the second positioning groove to the first positioning groove.

6. The syringe of claim 5, wherein a third inner sliding wall, which is matched with
the first bulge part, is arranged on the inside wall of the inner sliding sleeve;
the third inner sliding wall is located at one side of the first positioning groove toward the second positioning groove;
and the height of the third inner sliding wall decreases gradually from the first positioning groove to the second positioning groove;
and the angle between the first inner sliding wall and the inside wall of the inner sliding sleeve is greater than the angle between the third inner sliding wall and the inside wall of the inner sliding sleeve.

7. The syringe of claim 1, wherein, the syringe includes an outer sliding sleeve;
the outer sliding sleeve is embedded in and connected to the inner sliding sleeve, the outer sliding sleeve and the inner sliding sleeve can slide relatively to each other;
before the injection, the inner sliding sleeve and the outer sliding sleeve slide; and the inner sliding sleeve, the outer sliding sleeve and the needle holder are overlapped, to expose the injecting needle for the injection;
after the injection, the inner sliding sleeve and the outer sliding sleeve slide back, so that the injecting needle is packed into the inner sliding sleeve and the outer sliding sleeve.

8. The syringe of claim 7, wherein a second bulge part is arranged on the head end of an outside wall of the inner sliding sleeve;
a third positioning groove and a fourth positioning groove, which are matched with
the second bulge part, are arranged on the outer sliding sleeve;
the third positioning groove is arranged on the head end of an inside wall of the outer sliding sleeve;
the fourth positioning groove is arranged on the tail end of the inside wall of the outer sliding sleeve.

9. The syringe of claim 8, wherein a second guiding groove, which extends along the outer sliding sleeve, is arranged on the inside wall of the outer sliding sleeve;
a second guiding bulgy part, which is matched with the second guiding groove, is arranged on the outside wall of the inner sliding sleeve.

10. The syringe of claim 9, wherein a second slope surface, which is matched with the second bulge part, is arranged on the edge of the tail end of the inside wall of the outer sliding sleeve.

11. The syringe of claim 9, wherein a second inner sliding wall, which is matched with
the second bulge part, is arranged on the inside wall of the outer sliding sleeve;
the second inner sliding wall is located at one side, towards the third positioning groove, of the fourth positioning groove;
and the height of the second inner sliding wall decreases gradually from the fourth positioning groove to the third positioning groove.

12. The syringe of claim 11, wherein a fourth inner sliding wall, which is matched with
the second bulge part, is arranged on the inside wall of the outer sliding sleeve;
the fourth inner sliding wall is located at one side, towards the forth positioning groove, of the third positioning groove;
and the height of the fourth inner sliding wall decreases gradually from the third positioning groove to the fourth positioning groove;
and the angle between the second inner sliding wall and the inside wall of the outer sliding sleeve is greater than the angle between the fourth inner sliding wall and the inside wall of the outer sliding sleeve.

13. The syringe of anyone of claims 1-12, wherein, the syringe includes a piston, where a liquid-guiding channel is arranged, the liquid-guiding channel comprises an injecting channel, liquid-diverging hole, and a guiding channel, successively; the piston is inserted into the chamber of the needle holder.
